# EUROPEAN PATENT APPLICATION

(11) **EP 4 534 645 A1**
(43) Date of publication of application: **09.04.2025**
(21) Application number: 23904775.6
(22) Date of filing: 31.10.2023
(51) Int. Cl.: C12N 1/16, A23L 7/104, A21D 8/04, C12N 1/04, C12R 1/645

(54) **YEAST, YEAST POWDER AND USE THEREOF IN COOKED WHEATEN FOOD PROCESSING**

(30) Priority: 23.08.2023 CN 202311065243
(71) Applicant: Shenzhen TaiSu Biotechnology Co., Ltd., Shenzhen, Guangdong (CN)
(72) Inventor: YE, Jinrong, Shenzhen, Guangdong 518120 (CN)
(74) Representative: Kurig, Thomas
(86) International application number: PCT/CN2023/128416
(87) International publication number: WO 2025/039369

(57) **Abstract**

The present invention discloses a microzyme, a microzyme powder and an application thereof in pasta processing. The microzyme is CDLB-YE05 *Kazachstania servazzii,* which is preserved at Institute of Microbiology, Chinese Academy of Sciences on July 17, 2023 with a preservation number of CGMCC NO.27948 and a preservation address of Building 3, Courtyard 1, Beichen West Road, Chaoyang District, Beijing. The microzyme powder is prepared by using the CDLB-YE05 *Kazachstania servazzii* provided by the present invention, and the microzyme powder is used for pasta processing so that polysaccharide compounds produced by reproduction and metabolism of the microzyme in a dough are increased, the stickiness of food is increased and the pasta is more tenacious, strong in fragrance and better in taste.

## Description

### Field of invention

The present invention belongs to the technical field of microorganisms, and particularly relates to a microzyme, a microzyme powder and an application thereof in pasta processing.

### Background art of the invention

Pasta is a favorite food of Chinese people. For thousands of years, our ancestors have made many special pastas with local flavors and tastes by hands in various places, and the special pastas are very tenacious and delicious. Some pastas with regional characteristics have become the local traditional food. However, with the progress of industry, in the modern society, it is difficult to use machines to make the unique taste and flavor of the traditional pasta in different places. The reason is that there are differences in microzyme species in different regions and different people. Different microzyme species are inoculated into the dough, and different metabolites will be produced during the metabolism of fermented starch, such as carbon dioxide, organic acid, alcohol, alcohol compounds, carbohydrates and ester compounds. These metabolites promote the produced pastas present different flavors. The more the carbohydrates are, the more sticky the starch is, the more lipid compounds are, the more fragrant the noodles are and the richer the taste is. This is also the main reason for the tenacity and rich tastes of noodles. Making of pastas by a machine has no yeast fermentation technology, and thus the unique taste of the traditional pasta in various places cannot be made.

Therefore, it is urgent to provide a novel microzyme capable of producing carbohydrates and lipid compounds to improve the tenacity and flavor of flour food.

### Disclosure of the invention

The purpose of the present invention is to provide a microzyme, a microzyme powder and an application thereof in pasta processing, which solves the problem that various pastas produced by the existing technology is not tenacious enough and has poor taste.

In order to achieve the above purpose, a first technical solution of the present invention is realized as follows: a microzyme is provided; the microzyme is CDLB-YE05 *Kazachstania servazzii,* which is preserved at Institute of Microbiology, Chinese Academy of Sciences on July 17, 2023 with a preservation number of CGMCC NO.27948 and a preservation address of Building 3, Courtyard 1, Beichen West Road, Chaoyang District, Beijing.

Further, a nucleotide sequence of the microzyme is shown as SEQ No.1.

A second technical solution of the present invention is realized as follows: a microzyme powder prepared by the microzyme is provided; the microzyme powder is obtained by mixing, freeze-drying and grinding a microzyme culture, skim milk powder and sorbitan monostearate; 60 g-80 g of the skim milk powder and 9 g-10 g of the sorbitan monostearate are added to each kilogram of the microzyme culture;

The microzyme culture is cultured from the microzyme.

Further, water of the microzyme powder is not greater than 10%, protein is not less than 4%, ash is not greater than 10%, and a number of the microzymes is not less than 10⁷ CFU.

Further, the microzyme culture is prepared by a method below:
activating the microzyme for 16 h-48 h, and then inoculating into a mixed medium, and after being uniformly stirred with water, culturing under aerobic conditions of 15°C-35°C for 20 h-72 h to obtain the microzyme culture.

Further, the mixed medium is prepared by a method below:
weighing 720 parts to 880 parts of malt, 90 parts to 110 parts of soybeans, and 90 parts to 110 parts of glutinous rice according to mass parts;
roasting the weighed substances at 120°C for 2 h, and then crushing and mixing to obtain the mixed medium.

A third technical solution of the present invention is realized as follows: an application of the microzyme powder in pasta processing is provided.

Further, a specific method is:
S1, according to a mass ratio, weighing 0.08-0.1 of microzyme powder, 100-110 of wheat flour, 28-30 of water, 0.1-0.15 of edible salt and 0.1-0.15 of edible alkali respectively;
S2, mixing the weighed microzyme powder, wheat flour, edible salt, edible alkali and water to form a dough, and fermenting the dough for 3 h-6 h to prepare pasta.

Further, the pasta comprises noodles, dumplings, wontons and cakes.

Compared with the prior art, the present invention has the following beneficial effects:
The microzyme powder is prepared by using the microzyme provided by the present invention, and the microzyme powder is used for pasta processing so that polysaccharide compounds produced by reproduction and metabolism of the microzyme in flour are increased, the stickiness of flour food is increased and the flour food is more tenacious and better in taste.

### Description of the drawings

Fig. 1 shows a colony morphology of CDLB-YE05 *Kazachstania servazzii* on potato dextrose agar;
Fig. 2 shows a colony morphology of CDLB-YE05 *Kazachstania servazzii* on a malt extract medium;
Fig. 3 is a comparison diagram of content of polysaccharide compounds produced by different microzymes;
In Fig. 4: (a) is the colony morphology of the microzyme powder, (b) is the colony morphology of active dry yeast (high sugar type), (c) is the colony morphology of active dry yeast (low sugar type), and (d) is the colony morphology of *Saccharomyces ellipsoideus;*
Fig. 5 is a glucose standard curve;
Fig. 6 is a comparison diagram of concentrations of polysaccharide compounds in each group of samples in embodiment 4.

### Detailed description of the invention

To make the purpose, the technical solution and the advantages of the present invention more clear, the present invention will be further described below in detail in combination with specific embodiments. It should be understood that the specific embodiment described herein is only used for explaining the present invention, not used for limiting the present invention.

It should be clear in the description of the present invention that terms such as "perpendicular", "transverse", "longitudinal", "front", "rear", "left", "right", "upper", "lower", "horizontal", etc. indicate direction or position relationships shown based on the drawings, and are only intended to facilitate the description of the present invention rather than to mean that the indicated device or element must have a specific direction or position, and therefore, shall not be understood as a limitation to the present invention. It should be noted in the description of the present invention that, unless otherwise specifically regulated and defined, terms such as "installation," "connected," and "connecting" shall be understood in broad sense, and for example, may refer to fixed connection or detachable connection or integral connection, and may refer to direct connection or indirect connection through an intermediate medium. For those ordinary skilled in the art, the specific meanings of the above terms in the present invention may be understood according to specific conditions.

### Embodiment 1

The microzyme is isolated and purified, and the obtained microzyme is identified.

### 1) Sample source: Lanzhou stretched noodle dough

In all kinds of pastas, as a regional food with unique local characteristics, Lanzhou stretched noodles are particularly tenacious and have a unique taste and fragrance due to long handmade time and increased inoculation number and variety of microzyme species in the dough.

### 2) Culture medium: potato dextrose agar (PDA) and malt extract medium

Specific components of potato dextrose agar per liter are: 300.0 g of potato (from which leached powder is extracted), 20.0 g of glucose, 15.0 g of agar and 0.1 g of chloramphenicol.

Specific components of the malt extract medium per liter are: 130.0 g of malt extract powder and 0.1 g of chloramphenicol.

### 3) Used reagents and instruments

**Table 1 List of Used Reagents and Instruments in Embodiment 1**

| Reagents/Instruments | Source |
|---|---|
| Phenol | Nanjing Chemical Reagent Co., Ltd. |
| Sulphuric acid | Nanjing Chemical Reagent Co., Ltd. |
| Sodium chloride | Tianjin Damao Chemical Reagent Factory |
| Potassium dichromate | Tianjin Baishi Chemical Industry Co., Ltd. |
| Phosphate buffer | Guangdong Huankai Biotechnology Co., Ltd. |
| FA2204B electronic analytical balance | Shanghai Yoke Instrument Co., Ltd. |
| DSX-280B portable mode steam sterilizer | Shanghai Shenan Medical Apparatus Factory |
| SW-CJ-1FD vertical clean bench | Suzhou Bolaier Purification Equipment Co., Ltd. |
| SPX-250 biochemical incubator | Shanghai Shenxian Constant Temperature Equipment Factory |
| 723CRT visible spectrophotometer | Shanghai Yoke Instrument Co., Ltd. |
| ZD-85A digital display gas bath thermostatic oscillator | Changzhou Putian Instrument Manufacturing Co., Ltd. |
| H2-16 table model high speed centrifuge | Hunan Kecheng Instrument Equipment Co., Ltd. |
| MD55 WM1000 dialysis bag | Union Carbide Corporation (Viskase) |

### 4) Isolation of single strain

25 g of Lanzhou stretched noodle dough is weighed, 225 mL of stroke-physiological saline solution is added to make 1:10 sample homogenate, and the sample homogenate is diluted to sample homogenate with 10⁶ gradient by 10-fold increase.

In each dilution, 1 mL of sample homogenate is absorbed respectively and added into two plates, and 20 mL of potato dextrose agar cooled to 46°C is measured and injected into plates. After cooling and solidification, the plates are incubated in a fungus incubator at 28°C for five days.

The colony morphology is observed and single colonies are selected therefrom. The selected single colonies are continuously cultured on potato dextrose agar plates with stripes, and then the morphology of the single colonies is observed until different single strains are isolated.

### 5) Screening of anaerogenic strain

180 mL of malt extract medium is prepared, autoclaved at 115°C for 15 min, and then cooled and subpackaged into 18 sterile glass test tubes of 15 mL. The air in Durham tubes is emptied, and the Durham tubes are placed in each test tube with the openings facing down.

The single strains obtained in 4) are selected and inoculated into the malt extract medium of each test tube, and a breathable filtration membrane is added to each test tube for sealing. The single strains are subjected to shaking culture at 30±1°C and rotating speed of 160 r/min for 72 h. The turbidity rate and gas production of the strain growth are observed. The strains with fast turbidity rate and no gas production are selected.

### 6) Screening of strains without alcohol production

The anaerogenic strain screened in 5) is diluted to sample homogenate with 10⁶ gradient by 10-fold increase. In each dilution, 1 mL of sample homogenate is absorbed into two sterile plates respectively, and 20 mL of potato dextrose agar cooled to 46°C is measured and injected into plates. After cooling and solidification, the plates are incubated in a fungus incubator at 28°C for five days. The colony morphology is observed, and medium and large single colonies with smooth surfaces and neat edges are selected.

The above medium and large single colonies are inoculated into the malt extract medium, and subjected to shaking culture at 30±1°C and rotating speed of 160 r/min for 72 h to obtain a bacteria solution.

1 mL of bacteria solution is absorbed, 1 mL of potassium bichromate solution with molar concentration of 0.1 mol/L is added and shaken, and the color change of a mixed solution is observed.

Strains that do not change the color are selected to repeat the above screening steps until yeast strains that do not produce gas or alcohol are selected.

The yeast strains that do not produce gas or alcohol and the yeast strains that produce gas and alcohol obtained in the above process are simultaneously inoculated into the malt extract medium, and the reproduction and metabolism are observed. It is found that the yeast strains that do not produce gas or alcohol make the malt extract medium more viscous and have a rose flavor.

### 7) Purification and genome sequencing of yeast strains that do not produce gas or alcohol

100 mL of malt extract medium is prepared by a triangular flask, and autoclaved at 115°C for 15 min for later use.

A small amount of malt extract medium suspension is added to the yeast strains that do not produce gas or alcohol, and the suspension is poured into the malt extract medium in the triangular flask, sealed with a breathable filtration membrane, and subjected to shaking culture at 28±1°C and a rotating speed of 120 r/min for 48 h.

Bacteria precipitates obtained after centrifugation and washing of wort bacteria suspension are frozen by liquid nitrogen, put into a sample tube, and sent to Shenzhen Academy of Metrology & Quality Inspection for strain identification. The sample is identified as CDLB-YE05 *Kazachstania servazzii* by 18sRNA, and sent to BGI Wuhan Sequencing Center for genome sequencing. The nucleotide sequence of CDLB-YE05 *Kazachstania servazzii* is shown as SEQ No.1 and Table 2.

**Table 2 Nucleotide Sequence of CDLB-YE05 Kazachstania servazzii**

| Strain | Nucleotide Sequence |
|---|---|
| CDLB-YE 05 *Kazachsta nia servazzii* | ATCTGGTTGATCCTGCCAGTAGTCATATGCTTGTCTCAAAGATTAAGCCATG CATGTCTA AGTATAAGCAATTTATACAGTGAAACTGCGAATGGCTCATTAAATCAGTTAT CGTTTATT TGATAGTTCCTTTACTACATGGTATAACTGTGGTAATTCTAGAGCTAATACAT GCTTAAA ATCCCGACCTTTTGGAAGGGATGTATTTATTAGATAAAAAATCAATGTCTTC GGACTCTT TGATGATTCATAATAACTTTTCGAATCGCATGGCCTTGTGCCGGCGATGGTT CATTCAAA TTTCTGCCCTATCAACTTTCGATGGTAGGATAGTGGCCTACCATGGTTTCAA CGGGTAAC GGGGAATAAGGGTTCGATTCCGGAGAGGGAGCCTGAGAAACGGCTACCA CATCCAAGGAA GGCAGCAGGCGCGCAAATTACCCAATCCTAATACAGGGAGGTAGTGACAA TAAATAACGA TACAGGGCCCTTTCGGGTCTTGTAATTGGAATGAGTACAATGTAAATACCTT AACGAGGA ACAATTGGAGGGCAAGTCTGGTGCCAGCAGCCGCGGTAATTCCAGCTCCA ATAGCGTATA TTAAAGTTGTTGCAGTTAAAAGCTCGTAGTTGAACTTTGGGCCTGGTTGGC CGGTCTGGC TTTTTGCCACGTACTGGAATGCAACCGGGCCTTTCCTTCTGGCTAACCTTA GGCTCCTTG TGGGTCTTTGGCGAACCAGGACTTTTACTTTGAAAAAATTAGAGTGTTCAA AGCAGGCGT ATTGCTCGAATATATTAGCATGGAATAATGGAATAGGACGTTTGGTTCTATTT |
| | TGTTGGT TTCTAGGACCATCGTAATGATTAATAGGGACGGTCGGGGGCATCAGTATTC AATTGTCAG AGGTGAAATTCTTGGATTTATTGAAGACTAACTACTGCGAAAGCATTTGCC AAGGACGTT TTCATTAATCAAGAACGAAAGTTAGGGGATCGAAGATGATCAGATACCGTC GTAGTCTTA ACCATAAACTATGCCGACTAGGGATCGGGTGGTGTTTTTAATGACCCACTC GGCACCTTA CGAGAAATCAAAGTCTTTGGGTTCTGGAGTATGGTCGCAAGGCTGAAACT TAAAGGAATT GACGGAAGGGCACCACCAGGAGTGGAGCCTGCGGCTTAATTTGACTCAAC ACGGGGAAAC TCACCAGGTCCAGACACAATAAGGATTGACAGATTGAGAGCTCTTTCTTGA TTTTGTGGG TGGTGGTGCATGGCCGTTCTTAGTTGGTGGAGTGATTTGTCTGCTTAATTGC GATAACGA ACGAGACCTTAACCTACTAAATAGTGGTGCTAGCATTTGCTGGTTCTTCCA CTTCTTAGA GGGACTATCGATTTCAAGTCGATGGAAGTTTGAGGCAATAACAGGTCTGTG ATGCCCTTA GACGTTCTGGGCCGCACGCGCGCTACACTGACGGAGCCAGCGAGTCTAAC CTAGGCCGAG AGGTCCTGGTAATCTTGTGAAACTCCGTCGTGCTGGGGATAGAGCTTTGTA ATTTTTGCT CTTCAACGAGGAATTCCTAGTAAGCGCAAGTCATCAGCTTGCGTTGATTAC GTCCTGCCC TTTGTACACACCGCCCGTCGCTAGTACCGATTGAATGGCTTAGTGAGGCCT CAGGATCTG CTAGAGAAGGGGCAACTCCATCTCAGAGCGGAAAATCTGGTCAAACTTGG TCATTTAGAG GAACTAAAAGTCGTAACAAGGTTTCCGTAGGTGAACCTGCGGAAGGATCA TTA |

### 8) Colony morphology of CDLB-YE05 Kazachstania servazzii

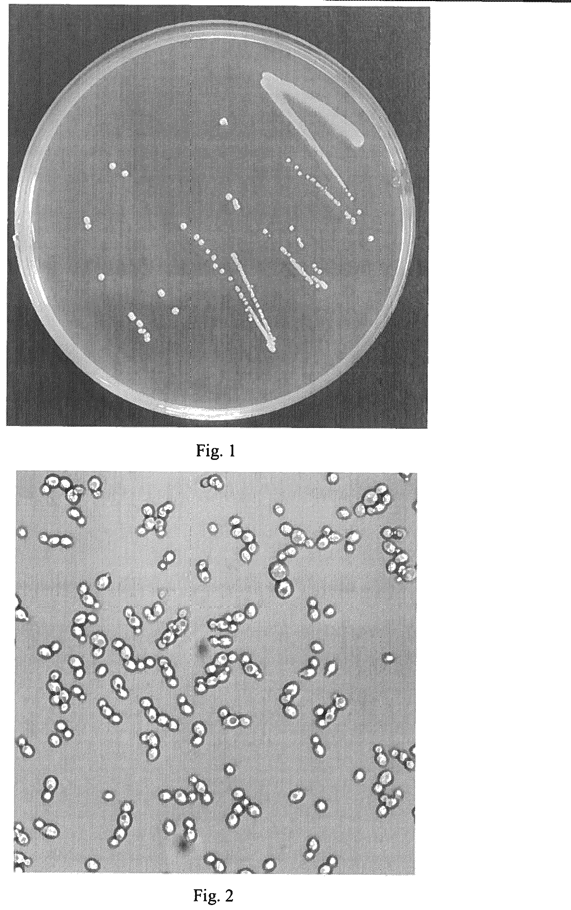

CDLB-YE05 *Kazachstania servazzii* is inoculated on a potato dextrose agar plate and cultured at 28°C for 5 days. The colony morphology is observed. As shown in Fig. 1, the colony of CDLB-YE05 *Kazachstania servazzii* is milky white, round, smooth in surface, opaque, non-reflective and neat in edges.

CDLB-YE05 *Kazachstania servazzii* is inoculated in a malt extract medium and cultured at 30°C for 2 days. The thalli of CDLB-YE05 *Kazachstania servazzii* is turgid, has no mycetome, does not produce a film, and has ester aroma and no bad odor. The cell morphology of CDLB-YE05 *Kazachstania servazzii* is observed under a microscope. As shown in Fig. 2, the cell morphology is a cleistothecium.

### 9) Genome characteristics of CDLB-YE05 Kazachstania servazzii

CDLB-YE05 *Kazachstania servazzii* has a genome, and the total length of a genome sequence is 13,468,544 bp. 5,415 genes are included, GC content accounts for 34.25%, gene length accounts for 66.81% of the total length of the genome, and 6,260 exons account for 64.51% of the total length of the genome. There are 5,415 CDS regions, accounting for 64.51% of the total length of the genome. 845 introns account for 2.31% of the total length of the genome. 1,046 reads of various types of non-coding RNA (ncRNA) copies account for 0.8106% of the total length of the genome, and 2,034,046 bp of various types of repeated sequences account for 15.1022% of the total length of the genome.

### 10) Culture preservation of CDLB-YE05 Kazachstania servazzii

Single colonies of CDLB-YE05 *Kazachstania servazzii* are selected by an inoculating loop in a sterile environment and inoculated into 250 mL of sterile wort. After uniform oscillation, the single colonies are expanded at a homothermal condition of 28±1°C for 48 h, and wort is selected for microscopy. The generation ofcleistothecia and the distribution of the colonies are observed in the colony morphology. If the visual field is full of ascospheric yeast, the expansion is successful; otherwise, the culture is continued and microscopy is performed every 2 h until the expansion is successful.

In the sterile environment, 17.5 g of skim milk powder is added into 250 mL of successfully expanded strain solution, mixed evenly and then freeze-dried. Freeze-dried powder is taken to detect the colony number of CDLB-YE05 *Kazachstania servazzii* greater than 10⁷ CFU as a preservation basis.

The freeze-dried powder of CDLB-YE05 *Kazachstania servazzii* is subpackaged into freeze-storage tubes with 0.1 g per tube, and then placed in vacuum packaging bags for vacuum sealing. After labeling, the freeze-dried powder is preserved at ultra low temperature of -80°C.

CDLB-YE05 *Kazachstania servazzii* is preserved at Institute of Microbiology, Chinese Academy of Sciences on July 17, 2023 with a preservation number of CGMCC NO.27948 and a preservation address of Building 3, Courtyard 1, Beichen West Road, Chaoyang District, Beijing.

### 11) Production of polysaccharide compounds of CDLB-YE05 Kazachstania servazzii

CDLB-YE05 *Kazachstania servazzii* and four gas-producing and alcohol-producing microzymes isolated from dough are inoculated in the malt extract medium and cultured for 24 h. 10 mL of wort fermentation broth fermented for 0 h, 4 h, 8 h, 12 h, 16 h, 20 h and 24 h are measured respectively.

The wort fermentation broth is centrifuged at 3500 r/min for 30 min, the thallus is discarded, and 1.5 g of trichloroacetic acid is added to the resulting supernatant, and then suspended evenly to stand at 4°C for 1 h.

The suspension is centrifuged at 11000 r/min for 1 min; protein precipitates are discarded; the resulting supernatant is transferred to a dialysis bag with a length of about 15 cm; and the dialysis bag is put into a beaker containing distilled water. Dialysate is replaced after dialysis at 4°C for 1 h, the dialysate is replaced again after continuing for dialysis for 2 h, and the dialysis is continued for 3 h.

After the end of the dialysis, the dialysate is discarded; the solution in the dialysis bag is transferred to a 100 mL volumetric flask; and water is added to a constant volume to obtain an experimental sample.

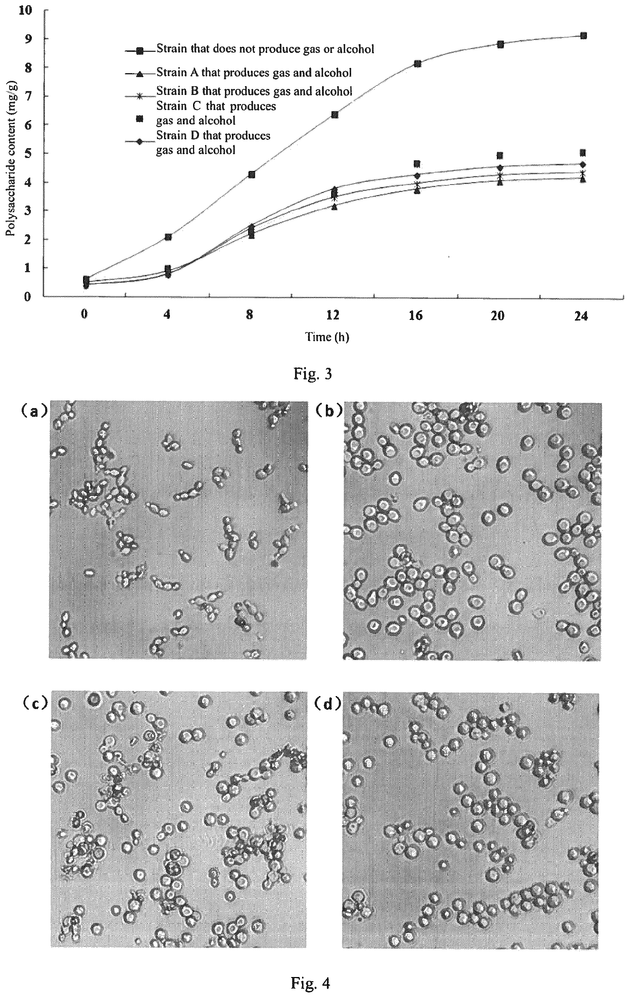

The experimental sample is diluted into different gradients. The absorbance is measured at 490 nm. The concentrations of polysaccharide compounds in different experimental samples are calculated according to a glucose standard curve and a regression equation. Detection results are shown in Fig. 3. Compared with other four gas-producing and alcohol-producing microzymes isolated in the dough, CDLB-YE05 *Kazachstania servazzii* can produce more polysaccharide compounds when fermentation in the malt extract medium.

### Embodiment 2

A mixed medium and a microzyme culture are prepared through the following steps.

### 1) Preparation of the mixed medium:

720-880 g of malt, 90-110 g of soybeans, and 90-110 g of glutinous rice are weighed, and the substances are roasted at 120°C for 2 h, and then crushed and mixed uniformly to obtain the mixed medium.

### 2) Preparation of a microzyme culture

After the freeze-dried powder of CDLB-YE05 *Kazachstania servazzii* obtained in embodiment 1 is activated in the malt extract medium for 16-48 h, the freeze-dried powder is added to the mixed medium at 1% inoculation amount (w/m; 800 g-1000 g of mineral water is added to the mixed medium per kg, and stirred evenly. Sterile air is introduced at a flow rate of 1 L/min. The mixture is cultured at 15°C-35°C under aerobic conditions for 20 h-72 h to obtain the microzyme culture.

CDLB-YE05 *Kazachstania servazzii* can grow and reproduce in the mixed medium, and can decompose and convert macromolecular proteins and starch particles in the mixed medium to produce ester aromatic compounds and polysaccharide compounds. After culture for 20 h-72 h, the produced ester aromatic compounds, polysaccharide compounds and medium components constitute a microzyme culture. The total acidity is not more than 30 mL/100 g, a viable cell rate is not less than 55%, and the content of the polysaccharide compounds is measured as not less than 1 mg/mL by a phenol-sulfuric acid method.

The following embodiments 3-5 relate to the preparation of microzyme powder, specifically as follows:

### Embodiment 3

The microzyme powder is prepared by using the microzyme culture obtained in embodiment 2, and a specific process is as follows:
70 g of skim milk powder and 9.5 g of sorbitan monostearate are added to 1000 g of microzyme culture, mixed evenly, then freeze-dried and ground to obtain the microzyme powder, wherein water ≤ 10%, protein ≥ 4%, ash ≤ 10% and microzyme number ≥ 10⁷ CFU.

### Embodiment 4

The microzyme powder is prepared by using the microzyme culture obtained in embodiment 2, and a specific process is as follows:
60 g of skim milk powder and 9 g of sorbitan monostearate are added to 1000 g of microzyme culture, mixed evenly, then freeze-dried and ground to obtain the microzyme powder.

### Embodiment 5

The microzyme powder is prepared by using the microzyme culture obtained in embodiment 2, and a specific process is as follows:
80 g of skim milk powder and 10 g of sorbitan monostearate are added to 1000 g of microzyme culture, mixed evenly, then freeze-dried and ground to obtain the microzyme powder.

The following embodiments 6-9 are embodiments of application of the microzyme powder in pasta processing, specifically as follows:

### Embodiment 6

The microzyme powder prepared in embodiment 3 is used in pasta processing to improve the taste and flavor of noodles and make the noodles more tenacious, and a specific method is as follows:
1 g of microzyme powder prepared in embodiment 3 and 1 kg of flour are weighed; and 280 g of water, 1 g of edible salt and 1 g of edible alkali are added, kneaded and proofed at 20°C-26°C for 4 h and 27°C-30°C for 3 h, respectively. After processing the proofed dough into noodles, the tensile breaking force of cooked noodles is tested. When the cross section of the cooked noodles is greater than 2 mm*2 mm, the tensile breaking force is not less than 15 g.

### Embodiment 7

The addition amount of wheat flour in embodiment 6 is adjusted to 1.1 kg, and other technological steps are the same as those in embodiment 6.

### Embodiment 8

0.8 g of microzyme powder prepared in embodiment 3 and 1 kg of flour are weighed; and 300 g of water, 1.5 g of edible salt and 1.5 g of edible alkali are added, kneaded and proofed at 20°C-26°C for 4 h and 27°C-30°C for 3 h, respectively. After processing the proofed dough into noodles, the tensile breaking force of cooked noodles is tested.

### Embodiment 9

The addition amount of wheat flour in embodiment 8 is adjusted to 1.1 kg, and other technological steps are the same as those in embodiment 8.

Embodiment 10 is a contrast experiment of application of the microzyme powder and other common microzymes in pasta, specifically as follows:

### Embodiment 10

After adding the microzyme powder prepared in embodiment 3 and other common microzymes to flour, the reproduction state of the colonies, the content change of the polysaccharide compounds and the tensile breaking force of the noodles in a fermentation process are detected to judge whether the noodles are tenacious or not.

### 1) Selected microzyme species and sources thereof

**Table 3 Selected Microzyme Species**

| Serial No. | Species Names | Source |
|---|---|---|
| 1 | Microzyme powder | Shenzhen Peptide Biotechnology Co., Ltd. |
| 2 | Active dry yeast (high sugar type) | Angel Yeast Co., Ltd. |
| 3 | Active dry yeast (low sugar type) | Angel Yeast Co., Ltd. |
| 4 | *Saccharomyces ellipsoideus* | Angel Yeast Co., Ltd. |

### 2) Culture medium: potato dextrose agar (PDA) and malt extract medium

Specific components of potato dextrose agar per liter are: 300.0 g of potato (from which leached powder is extracted), 20.0 g of glucose, 15.0 g of agar and 0.1 g of chloramphenicol.

Specific components of the malt extract medium per liter are: 130.0 g of malt extract powder and 0.1 g of chloramphenicol.

### 3) Reagents and raw material

**Table 4 List of Selected Reagents**

| Reagents | Source |
|---|---|
| Phenol | Nanjing Chemical Reagent Co., Ltd. |
| Sulphuric acid | Nanjing Chemical Reagent Co., Ltd. |
| Glucose | Shanghai Aladdin Biochemical Technology Co., Ltd. |
| Wonder Farm wheat flour | Yihai Kerry Arawana Holdings Co., Ltd. |
| Yueyan edible salt | Guangdong Salt Industry General Company |
| Gusong edible alkali | Tianjin Hongyi Food Manufacturing Co., Ltd. |

### 4) Experimental equipment and instruments

**Table 5 List of Selected Instruments**

| Instruments | Source |
|---|---|
| FA2204B electronic analytical balance | Shanghai Yoke Instrument Co., Ltd. |
| DSX-280B portable mode steam sterilizer | Shanghai Shenan Medical Apparatus Factory |
| SW-CJ-1FD vertical clean bench | Suzhou Bolaier Purification Equipment Co., Ltd. |
| SPX-250 biochemical incubator | Shanghai Shenxian Constant Temperature Equipment Factory |
| 723CRT visible spectrophotometer | Shanghai Yoke Instrument Co., Ltd. |
| ZD-85A digital display gas bath thermostatic oscillator | Changzhou Putian Instrument Manufacturing Co., Ltd. |
| H2-16 table model high speed centrifuge | Hunan Kecheng Instrument Equipment Co., Ltd. |
| OLYMPUS-CX31 optical microscope | Olympus (Shenzhen )Industrial Ltd. |
| DHG-9420B electro-thermostatic blast oven | Shanghai Shenxian Constant Temperature Equipment Factory |
| F-031 SD ultrasonic washer | Shenzhen Fuyang Technology Group Co., Ltd. |
| MD55 WM1000 dialysis bag | Union Carbide Corporation (Viskase) |
| DTB-30g tensiometer | Shenzhen Xindeya Precision Instrument Co., Ltd. |
| XK97-A colony counter | Hangzhou Qiwei Instrument Co., Ltd. |
| Model 220 750w noodle press | Henan Bangqiao Weili Trading Co., Ltd. |

### 5) Experimental process

### 5.1 Preparation of yeast strain solution

### 5.1.1 Strain activation

The microzyme powder prepared in embodiment 3, active dry yeast (high sugar type), active dry yeast (low sugar type) and *Saccharomyces ellipsoideus* are weighed respectively, inoculated into 10 mL of malt extract medium according to the inoculation amount of 0.1% (w/v), and subjected to aerobic fermentation at 30°C for 24 h.

### 5.1.2 Strain microscopy

Microscopy is performed on four activated yeast strains, as shown in Fig. 4. In Fig. 4, (a) is the colony morphology of the microzyme powder, (b) is the colony morphology of active dry yeast (high sugar type), (c) is the colony morphology of active dry yeast (low sugar type), and (d) is the colony morphology of *Saccharomyces ellipsoideus.*

### 5.1.3 Adjustment of colony number of strain solution

In order to ensure the consistency of experimental conditions, the colony number in the activated different yeast strain solutions is uniformly adjusted, and specific steps are as follows:
A spectrophotometer is zeroed by fresh wort to determine the OD600 value of different strain solutions, the fresh wort is used for gradual dilution, the OD600 value is adjusted to 0.5, and the corresponding strain solution concentration is about 10⁷ CFU/mL.

### 5.2 Manufacturing of glucose standard curve

50 mg of standard glucose is weighed into a volumetric flask; an appropriate amount of distilled water is added to a scale of 500 mL; the solution is shaken well to prepare a standard glucose solution of 100 µg/mL; then the standard glucose solution, the distilled water, 5% phenol solution and concentrated sulfuric acid are added as shown in Table 6, shaken well, heated in a boiling water bath for 30 min, taken out, shaken well, cooled and placed at room temperature for 20 min. The absorbance is measured at 490 nm.

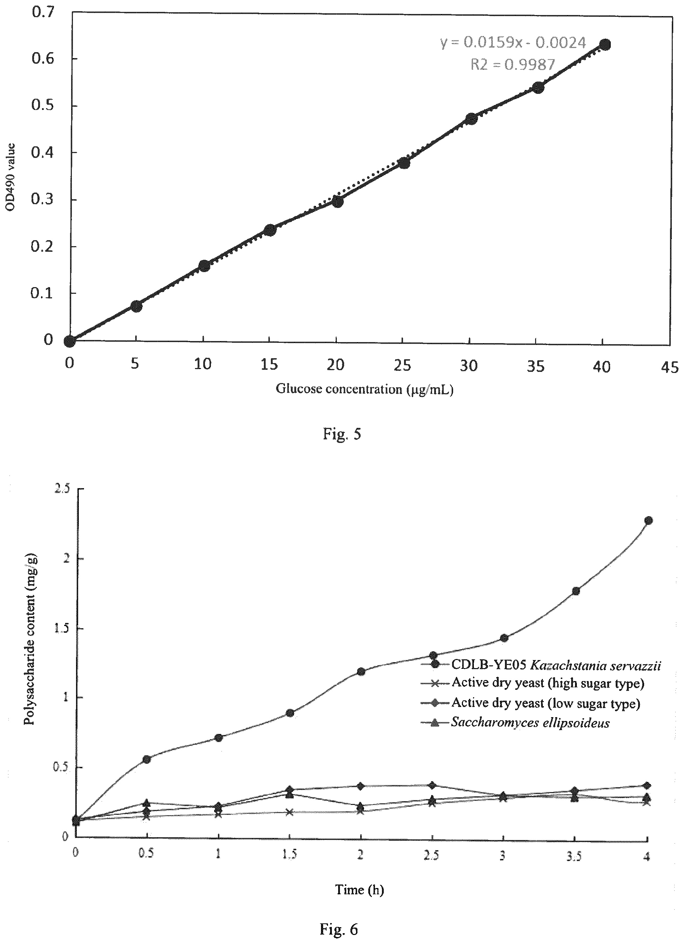

A standard curve is drawn with the glucose concentration as an abscissa and the absorbance as an ordinate, and a regression equation is solved, as shown in Fig. 5.

**Table 6 Formulations of Reagents for Manufacturing of Glucose Standard Curve**

| Reagents | Test Tube No. | | | | | | | | |
|---|---|---|---|---|---|---|---|---|---|
| | 1 | 2 | 3 | 4 | 5 | 6 | 7 | 8 | 9 |
| Standard glucose solution (mL) | 0 | 0.05 | 0.1 | 0.15 | 0.2 | 0.25 | 0.3 | 0.35 | 0.4 |
| Distilled water (mL) | 1 | 0.95 | 0.9 | 0.85 | 0.8 | 0.75 | 0.7 | 0.65 | 0.6 |
| 5% phenol solution (mL) | 1.6 | 1.6 | 1.6 | 1.6 | 1.6 | 1.6 | 1.6 | 1.6 | 1.6 |
| Concentrated | 7 | 7 | 7 | 7 | 7 | 7 | 7 | 7 | 7 |
| sulfuric acid (mL) | | | | | | | | | |

### 5.3 Inoculation of microzyme in flour for fermentation

Various ingredients are added according to the ratio in Table 7. There are five groups, wherein for each group, 500 g of flour, 1 g of edible salt and 1 g of alkali are weighed respectively, and 5 mL of adjusted yeast strain solutions in groups A, B, C and D and blank in group E are measured and added to 140 mL of drinking water and mixed evenly. Then, drinking water containing yeast is slowly added to flour and stirred evenly for half an hour for fermenting into a dough at 30°C for 4 h.

**Table 7 Mixing Ratio of Yeast Strain Solutions and Flour**

| Groups | Species Names | Strain solution volume (mL) | Flour (g) | Mineral water (mL) | Edible salt (g) | Edible alkali (g) |
|---|---|---|---|---|---|---|
| A | Microzyme powder | 5 | 500 | 140 | 1 | 1 |
| B | Active dry yeast (high sugar type) | 5 | 500 | 140 | 1 | 1 |
| C | Active dry yeast (low sugar type) | 5 | 500 | 140 | 1 | 1 |
| D | *Saccharomyces ellipsoideus* | 5 | 500 | 140 | 1 | 1 |
| E | Blank | 0 | 500 | 140 | 1 | 1 |

### 5.4 Extraction of polysaccharide compounds produced by flour fermentation

### Specific steps are as follows:

In the fermentation process of flour, 3 g of samples are taken every 0.5 h. After the samples are spread out evenly, the samples are dried to semi-dry by blowing at 50°C, and then the temperature is gradually increased to 80°C until the samples are agglomerated. The samples are cooled, then crushed and screened with a sieve diameter of 0.9 mm. The sieved samples are mixed evenly and put into a wide-mouth bottle for sealing storage.

1.0 g of sieved samples are weighed and placed in a 50 mL centrifuge tube with stopper. The samples are infiltrated with 5 mL of water, and 20 mL of absolute ethyl alcohol is slowly added and fully oscillated to obtain a uniform mixture.

The mixture is ultrasonically extracted for 30 min, and centrifuged for 10 min, and then insolubles are washed with 10 mL of ethyl alcohol and centrifuged.

The above insolubles are transferred to a conical flask with cover by 30 mL of water successively, sealed with a breathable sealing membrane, extracted in a boiling water bath for 2 h, and cooled to room temperature, and filtrate is filtered for use.

The filtrate is transferred to a dialysis bag with a length of about 15 cm; and the dialysis bag is put into a 1000 mL beaker containing distilled water. Dialysate is replaced after dialysis at 4°C for 1 h, the dialysate is replaced again after continuing for dialysis for 2 h, and finally the dialysate is replaced again after the dialysis is continued for 3 h.

After the end of the dialysis, the dialysate is discarded; the solution in the dialysis bag is transferred to a 100 mL volumetric flask; and water is added to a constant volume to obtain an experimental sample.

### 5.5 Determination of polysaccharide compounds

The experimental sample prepared in 5.4 is taken and diluted into different gradients. The absorbance is measured at 490 nm. The concentrations of polysaccharide compounds in the experimental samples are calculated with reference to a glucose standard curve and a regression equation. Results are shown in Fig. 6. After the microzyme powder prepared in embodiment 3 is added, the content of polysaccharide produced by fermentation in the dough is gradually increased with the extension of fermentation time. Since the content of polysaccharide is directly proportional to the viscosity of the noodles, the tensile breaking force of the noodles is increased and the noodles are more tenacious.

### 5.6 Determination of number of microzymes

The viable counts of microzymes in the experimental samples fermented for 0.5 h and 4 h are determined by a plate count method respectively, and a medium used for detection is potato dextrose agar.

Detection results are shown in Table 9. The microzyme powder prepared in embodiment 3 has the highest reproduction speed during dough fermentation.

### 5.7 Determination of tensile breaking force of noodles produced by samples

The doughs of different treatment groups after fermentation for 4 are rolled, compounded and slivered according to a unified standard. The cross-section size of the noodles is 3.5 mm*3.5 mm. The noodles are boiled for 150s and then cooled with cold water. The fixed length of both ends of the noodles is 10 cm. The tensile breaking force value of the noodles when pulled off is measured with a tensioner. Different treatment groups are measured for 35 times respectively, and the original data is recorded, as shown in Table 8. The average value of the remaining data after the highest and lowest values are removed from each group is calculated, as shown in Table 9.

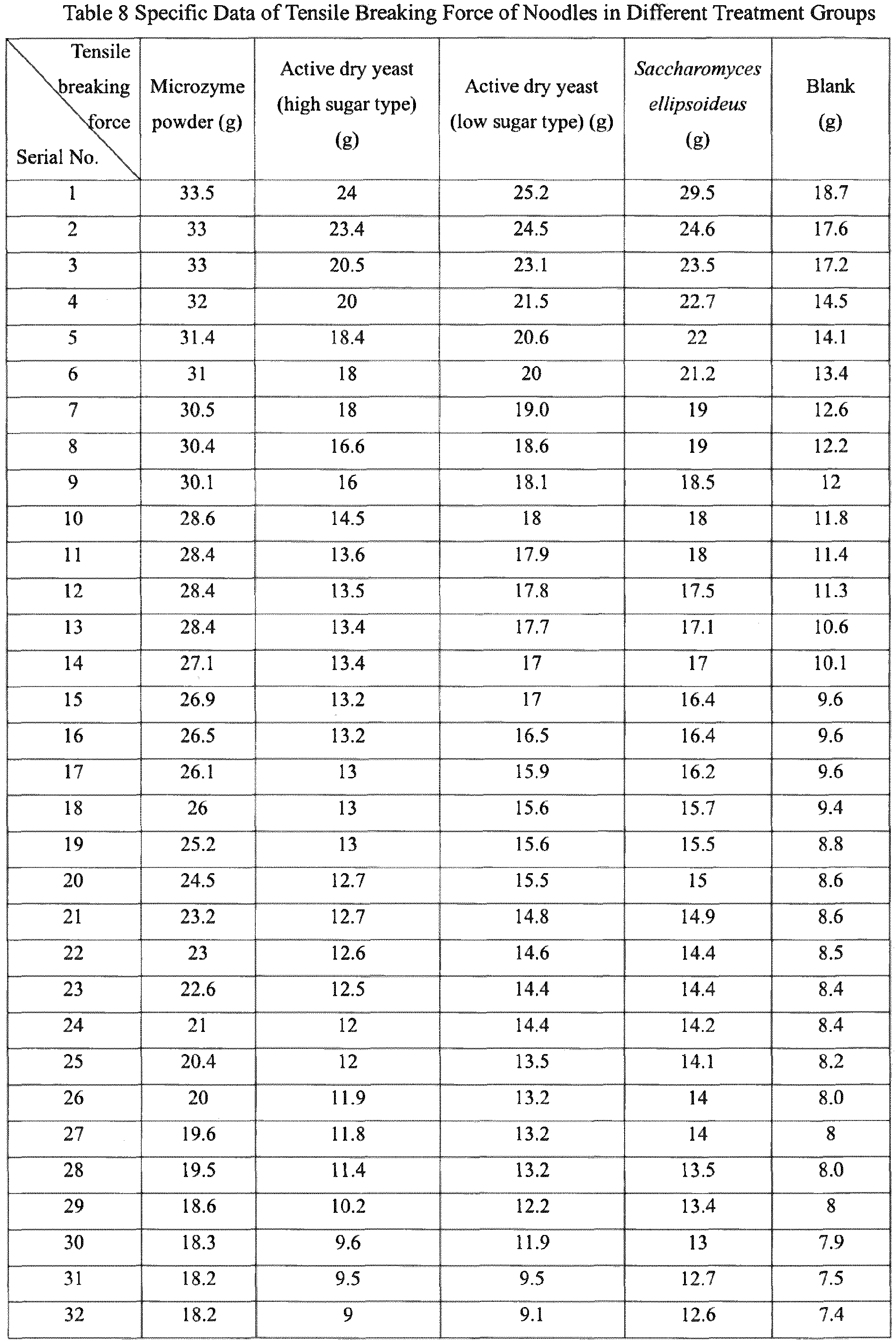

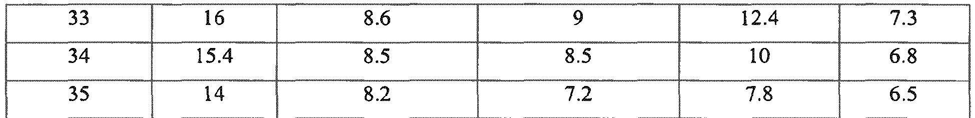

**Table 9 Average Value of Tensile Breaking Force of Noodles in Each Treatment Group**

| Grou ps | Sample Group | Number of Microzymes after 0.5 h (CFU/g) | Number of Microzymes after 4 h (CFU/g) | Highest Tensile Breaking Force Value (g) | Lowest Tensile Breaking Force Value (g) | Average Value (g) |
|---|---|---|---|---|---|---|
| A | Microzyme powder | 3.5×10⁵ | 1.4×10⁶ | 33.5 | 14 | 24.9 |
| B | Active dry yeast (high sugar type) | 9.2×10⁵ | 8.8×10⁵ | 24 | 8.2 | 13.6 |
| C | Active dry yeast (low sugar type) | 8.7×10⁵ | 7.5×10⁵ | 25.2 | 7.2 | 15.8 |
| D | *Saccharom yces ellipsoideu s* | 6.8×10⁵ | 1.2×10⁵ | 29.5 | 7.8 | 16.4 |
| E | Blank | 0 | 0 | 18.7 | 6.5 | 10.2 |

In conclusion, when the microzyme type in the processing formula of wheat pasta is changed by a single factor, the metabolites of the microzyme in the dough will be changed. The microzyme powder provided by the present invention produces a large number of polysaccharide compounds in the metabolic process. Since the content of polysaccharide compounds in the dough is directly proportional to the viscosity, addition of the above microzyme powder in pasta processing can improve the tenacity and taste of pasta.

The above only describes preferred specific embodiments of the present invention, but the protection scope of the present invention is not limited thereto. Any change or replacement contemplated easily by those skilled in the art familiar with the technical field within the technical scope disclosed by the present invention shall be covered within the protection scope of the present invention. Therefore, the protection scope of the present invention should be determined by the protection scope of the claims.

## Claims

1. A microzyme, **characterized in that** the microzyme is CDLB-YE05 *Kazachstania servazzii,* which is preserved at Institute of Microbiology, Chinese Academy of Sciences on July 17, 2023 with a preservation number of CGMCC NO.27948 and a preservation address of Building 3, Courtyard 1, Beichen West Road, Chaoyang District, Beijing.

2. The microzyme according to claim 1, **characterized in that** a nucleotide sequence of the microzyme is shown as SEQ No.1.

3. A microzyme powder prepared by the microzyme according to claim 1 or 2, **characterized in that** the microzyme powder is obtained by mixing, freeze-drying and grinding a microzyme culture, skim milk powder and sorbitan monostearate; 60 g-80 g of the skim milk powder and 9 g-10 g of the sorbitan monostearate are added to each kilogram of the microzyme culture;
the microzyme culture is cultured from the microzyme.

4. The microzyme powder according to claim 3, **characterized in that** water of the microzyme powder is not greater than 10%, protein is not less than 4%, ash is not greater than 10%, and a number of the microzymes is not less than 10⁷ CFU.

5. The microzyme powder according to claim 3, **characterized in that** the microzyme culture is prepared by a method below:
activating the microzyme for 16 h-48 h, and then inoculating into a mixed medium, and after being uniformly stirred with water, culturing under aerobic conditions of 15°C-35°C for 20 h-72 h to obtain the microzyme culture.

6. The microzyme powder according to claim 5, **characterized in that** the mixed medium is prepared by a method below:
weighing 720 parts to 880 parts of malt, 90 parts to 110 parts of soybeans, and 90 parts to 110 parts of glutinous rice according to mass parts;
roasting the weighed substances at 120°C for 2 h, and then crushing and mixing to obtain the mixed medium.

7. An application of the microzyme powder according to any one of claims 3-6 in pasta processing.

8. The application of the microzyme powder in pasta processing according to claim 7, **characterized in that** a specific method is:
S1, according to a mass ratio, weighing 0.08-0.1 of microzyme powder, 100-110 of wheat flour, 28-30 of water, 0.1-0.15 of edible salt and 0.1-0.15 of edible alkali respectively;
S2, mixing the weighed microzyme powder, wheat flour, edible salt, edible alkali and water to form a dough, and fermenting the dough for 3 h-6 h to prepare pasta.

9. The application of the microzyme powder in pasta processing according to claim 8, **characterized in that** the pasta comprises noodles, dumplings, wontons and cakes.
